(19) Europäisches Patentamt / European Patent Office / Office européen des brevets

(11) **EP 3 409 230 B1**

(12) **EUROPÄISCHE PATENTSCHRIFT**

(45) Veröffentlichungstag und Bekanntmachung des Hinweises auf die Patenterteilung:
**22.05.2019   Patentblatt 2019/21**

(21) Anmeldenummer: **17173787.7**

(22) Anmeldetag: **31.05.2017**

(51) Int Cl.:
*A61B 34/30* (2016.01)    *A61B 8/00* (2006.01)
*A61B 34/32* (2016.01)    *G06N 3/02* (2006.01)
*A61B 90/00* (2016.01)    *A61B 34/10* (2016.01)
*A61B 34/20* (2016.01)

(54) **BEWEGEN EINES ROBOTERARMS**

MOVEMENT OF A ROBOT ARM

MOUVEMENT D'UN BRAS DE ROBOT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.12.2018   Patentblatt 2018/49**

(73) Patentinhaber: **Siemens Healthcare GmbH 91052 Erlangen (DE)**

(72) Erfinder:
• **Freudenberger, Jörg**
  **90562 Kalchreuth (DE)**
• **Haider, Sultan**
  **91052 Erlangen (DE)**
• **Molnar, Peter**
  **90403 Nürnberg (DE)**

(56) Entgegenhaltungen:
EP-A1- 2 514 366    US-A1- 2005 154 295
US-A1- 2007 021 738    US-A1- 2008 010 706
US-A1- 2009 088 639    US-A1- 2014 046 128

EP 3 409 230 B1

**Beschreibung**

Die Erfindung betrifft ein Verfahren zum Bewegen eines Roboterarms für eine Ultraschalluntersuchung, wobei an dem Roboterarm ein Ultraschallkopf befestigt ist, ein zugehöriges Ultraschallsystem und ein zugehöriges Computerprogrammprodukt.

Mit einem Ultraschallsystem kann üblicherweise ein zu untersuchender Körper einer zu untersuchenden Person, insbesondere eines Patienten, nicht-invasiv untersucht werden. Ein Ultraschallsystem für die medizinische Diagnostik weist üblicherweise einen Ultraschallkopf auf, welcher von einem Arzt auf eine Körperoberfläche des Patienten aufgesetzt wird und in engem Kontakt mit der Haut des Patienten ein Ultraschallbild erzeugt. Hierzu enthält der Ultraschallkopf ein eindimensionales oder zweidimensionales piezoelektrisches Array, in dem elektrische Sendepulse in Druckpulse mit einer bestimmten Frequenz oder in einem bestimmten Frequenzband bzw. Druckpulse in elektrische Empfangssignale umgewandelt werden. Von den elektrischen Empfangssignalen können normalerweise Ultraschallbilder generiert werden, wobei die Ultraschallbilder üblicherweise in einem bestimmten Mode dargestellt werden.

Üblicherweise wird während einer Ultraschalluntersuchung der Ultraschallkopf bewegt. In US 9,420,997 B2 ist ein Verfahren beschrieben, bei welchem Bewegungsartefakte bei einer Ultraschalluntersuchung unterdrückt werden können.

In WO 2017 020 081 A1 ist eine Kombination eines Roboterarms mit einem Ultraschallkopf offenbart.

US 2009 0088639 A1 offenbart eine Anordnung, wobei ein Ultraschallkopf an einem Roboterarm befestigt ist. Künstliche neuronale Netze sind bereits seit längerer Zeit im Interesse der Wissenschaft und Wirtschaft. Künstliche neuronale Netze haben als Vorbild die natürlichen neuronale Netze, welche von Nervenzellvernetzungen im Gehirn und Rückenmark gebildet werden. Ein künstliches neuronales Netz weist üblicherweise mehrere Knoten und Verbindungen zwischen Knoten auf. In einer Trainingsphase kann das neuronale Netz lernen, indem Gewichtungen der Verbindungen verändert werden. Künstliche neuronale Netze liefern üblicherweise bei herausfordernden Anwendungen bessere Ergebnisse als konkurrierende maschinelle Lernverfahren.

DE 10 2015 212 953 A1 beschreibt eine mögliche Anwendung eines trainierten künstlichen neuronalen Netzes.

Der Erfindung liegt die Aufgabe zu Grunde, ein Verfahren zum Bewegen eines Roboterarms für eine Ultraschalluntersuchung, wobei an dem Roboterarm ein Ultraschallkopf befestigt ist, ein zugehöriges Ultraschallsystem und ein zugehöriges Computerprogrammprodukt anzugeben.

Die Aufgabe wird durch die Merkmale der unabhängigen Ansprüche gelöst. Vorteilhafte Weiterbildungen sind in den abhängigen Ansprüchen angegeben.

Nachstehend wird die erfindungsgemäße Lösung der Aufgabe sowohl in Bezug auf das beanspruchte Ultraschallsystem und das Verfahren zum Bewegen des Roboterarms für eine Ultraschalluntersuchung als auch in Bezug auf das zugehörige Computerprogrammprodukt beschrieben. Hierbei erwähnte Merkmale, Vorteile oder alternative Ausführungsformen sind ebenso auf die anderen beanspruchten Gegenstände zu übertragen und umgekehrt. Mit anderen Worten können die gegenständlichen Ansprüche (die beispielsweise auf ein Ultraschallsystem gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet. Das erfindungsgemäße Verfahren zum Bewegen eines Roboterarms für eine Ultraschalluntersuchung, wobei an dem Roboterarm ein Ultraschallkopf befestigt ist, umfasst folgende Verfahrensschritte:

- Bereitstellen eines trainierten künstlichen neuronalen Netzes,
- Erfassen einer medizinischen Fragestellung,
- Bestimmen eines Bewegungsdatensatzes, welcher einen Bewegungsablauf des Roboterarms aufweist, durch Anwendung des trainierten künstlichen neuronalen Netzes auf die medizinische Fragestellung,
- Übertragen des Bewegungsdatensatzes an eine Steuereinheit des Roboterarms und
- Bewegen des Roboterarms gemäß dem Bewegungsablauf des Bewegungsdatensatzes, gekennzeichnet dadurch, dass der Bewegungsablauf mittels eines Projektors auf eine Körperoberfläche eines Patienten projiziert wird.

Das erfindungsgemäße Verfahren kann derart eine bessere und einfachere Ultraschalluntersuchung erlauben. Die Erfindung kann daher insbesondere mehrere Vorteile bieten:
Beispielsweise kann der Roboterarm einen Nutzer beim Bewegen unterstützen und/oder der Roboterarm wird automatisch bewegt. Dies ist insbesondere vorteilhaft, wenn beispielsweise ein Nutzer, insbesondere ein Arzt, bei der Ultraschalluntersuchung unterstützt werden muss, weil der Arzt unerfahren oder mit der Ultraschalluntersuchung nicht vertraut ist oder weil der Arzt gleichzeitig eine chirurgische Intervention durchführt. Ferner ist es möglich, dass dort, wo kein Arzt und damit kein mit der Ultraschalluntersuchung vertrauter Nutzer zur Durchführung bereitsteht, die Ultraschalluntersuchung automatisch oder sogar autonom durchgeführt wird, indem der Roboterarm automatisch bewegt wird. Vorzugweise ist keine Fernsteuerung des Roboterarms durch einen erfahrenen Ultraschallfacharzt notwendig. Außerdem ist die hohe Wiederholpräzision bei beiden Verfahren zum Bewegen des Roboterarms hervorzuheben, wodurch eine Kosteneinsparung bei der Ultraschalluntersuchung und/oder eine eigentlich Durchführung der Ultraschalluntersuchung überhaupt erst möglich ist. Auf Grund der hohen Wiederholpräzision ist der Roboterarm für die Ultraschalluntersuchung, wobei an dem Roboterarm der Ultraschallkopf befestigt ist, besonders vorteilhaft. Beispielsweise könnte dadurch ein Screening der Herzfunktion in einer großen Population mit einem hohen Automatisierungsgrad und kosteneffizient durchgeführt werden.

Ein künstliches neuronales Netz (KNN, englisch artificial neural network - ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen. Zwei unterschiedliche trainierte künstliche neuronale Netze können so, obwohl sie beispielsweise die gleiche Architektur und/oder Topologie aufweisen, unterschiedliche Aufgaben durchführen.

Ein Beispiel für ein künstliches neuronales Netz ist ein flaches künstliches neuronales Netz, welches normalerweise nur eine einzelne verdeckte Schicht zwischen der Eingabeschicht und der Ausgabeschicht enthält und damit relativ einfach zu trainieren ist. Ein weiteres Beispiel ist ein tiefes künstliches neuronales Netz, welches zwischen der Eingangsschicht und der Ausgabeschicht mehrere (beispielsweise bis zu zehn) verschachtelte verdeckte Schichten von künstlichen Neuronen enthält. Das tiefe künstliche neuronale Netz ermöglicht dabei eine verbesserte Erkennung von Mustern und komplexen Zusammenhängen. Weiterhin kann ein gefaltetes tiefes künstliches neuronales Netz für das Bestimmen eines Bewegungsdatensatzes gewählt werden, welches zusätzlich Faltungsfilter, beispielsweise Kantenfilter, einsetzt.

Es wird nun vorgeschlagen, dass ein derart ausgebildetes trainiertes künstliches neuronales Netz gewählt wird, wobei die Anwendung des trainierten künstlichen neuronalen Netzes auf die medizinische Fragestellung ein Bestimmen eines Bewegungsdatensatzes ermöglicht. Das trainierte künstliche neuronale Netz kann dabei lediglich zum Bestimmen des Bewegungsdatensatzes durch Anwendung des trainierten künstlichen neuronalen Netzes auf die medizinische Fragestellung geeignet sein. Alternativ kann es auch andere Aufgabe übernehmen. Es kann vorkommen, dass man verschiedene künstliche neuronale Netze aufsetzt, welche das Bestimmen des Bewegungsdatensatzes gleichermaßen durchführen können.

Im vorliegenden Verfahren wird insbesondere ein bereits trainiertes künstliches neuronales Netz für das Bestimmen des Bewegungsdatensatzes durch Anwendung des trainierten künstlichen neuronalen Netzes auf die medizinische Fragestellung bereitgestellt. Das Training des künstlichen neuronalen Netzes kann dabei mittels zumindest eines Trainingsbewegungsdatensatzes und mittels zumindest einer medizinischen Trainingsfragestellung durchgeführt worden sein. Verschiedene Möglichkeiten zum Training des künstlichen neuronalen Netzes sind in einem der folgenden Abschnitte beschrieben. Vorteilhafterweise kann das künstliche neuronale Netz mittels eines in einem der folgenden Abschnitte beschriebenen Verfahren zu einem Bereitstellen eines trainierten künstlichen neuronalen Netzes für ein Bestimmen eines Bewegungsdatensatzes, welcher einen Bewegungsablauf eines Roboterarms für eine Ultraschalluntersuchung aufweist, wobei an dem Roboterarm ein Ultraschallkopf befestigt ist, trainiert werden.

Das Erfassen der medizinischen Fragestellung kann aufweisen, dass ein Sensor, insbesondere eine Kamera, zumindest einen Parameter der medizinischen Fragestellung bereitstellt. Alternativ oder zusätzlich ist es auch denkbar, dass das Erfassen der medizinischen Fragestellung umfasst, dass mittels einer graphischen Benutzeroberfläche ein Nutzer zumindest einen Parameter der medizinischen Fragestellung bereitstellt. Grundsätzlich ist es auch denkbar, dass sowohl ein Sensor als auch ein Nutzer zumindest einen Parameter der medizinischen Fragestellung bereitstellen.

Der Sensor, insbesondere eine optische Kamera, kann vorzugweise dafür geeignet sein, eine zu untersuchende Person, insbesondere einen Patienten, bildlich zu erfassen. Mittels geeigneter und bekannter Algorithmen kann eine Bilderkennung durchgeführt werden. Vorzugweise kann der Sensor die zu untersuchende Person mehrmals bildlich erfassen, beispielsweise in einer Filmaufnahme. Die Bilderkennung kann auch auf die Filmaufnahme angewandt werden. Mittels der Bilderkennung kann zumindest ein Parameter der medizinischen Fragestellung erfasst werden, welche beispielsweise von einer Steuereinheit des Sensors bereitgestellt werden. Die medizinische Fragestellung kann zumindest einen mittels der Bilderkennung erfassten Parameter aufweisen. Grundsätzlich ist es auch denkbar, dass der Sensor mehrere Sensor-Komponenten aufweist, welche sich je in der Sensorik unterscheiden.

Der Nutzer kann beispielsweise auf einer graphischen Benutzeroberfläche zumindest einen Parameter der medizinischen Fragestellung bereitstellen. Dafür weist beispielsweise eine Planungseinheit einen Monitor auf, wobei der Monitor die graphische Benutzeroberfläche aufweist. Üblicherweise kann der Nutzer mit der graphischen Benutzeroberfläche mit einem Eingabegerät interagieren. Vorzugweise kann die graphische Benutzeroberfläche Standardwerte oder eine Liste mit üblichen Werten oder Parameter bereitstellen, welche der Nutzer einsehen und auswählen kann. Grundsätzlich ist es auch denkbar, dass der Nutzer zumindest einen Parameter der medizinischen Fragestellung frei eingeben oder auswählen kann. Der zumindest eine Parameter der medizinischen Fragestellung kann vollständig von dem Sensor, vollständig von dem Nutzer oder teilweise von dem Sensor und dem Nutzer bereitgestellt werden.

Die medizinische Fragestellung weist in einer weiteren Ausführung zumindest einen Parameter der folgenden Liste auf:

- eine zu untersuchende Körperregion eines Patienten,
- einen Mode der Ultraschalluntersuchung,
- eine Frequenz der Ultraschalluntersuchung und
- eine Vorgabe für die Ultraschalluntersuchung.

Die zu untersuchende Körperregion des Patienten kann einen Bereich oder einen Teil des Patienten aufweisen, der in der Ultraschalluntersuchung untersucht werden soll. Beispiele für die zu untersuchende Körperregion sind Abdomen, Thorax oder Hals. Grundsätzlich ist es auch denkbar, dass die zu untersuchende Körperregion sich auf Organe wie Leber, Herz, Gallenblase oder Schilddrüse des Patienten beziehen. Der Sensor kann üblicherweise lediglich eine Körperoberfläche des Patienten erfassen und mittels Bilderkennung beispielsweise die entsprechende Körperregion bestimmen. Für eine Lokalisierung der Organe des Patienten kann in bestimmten Fällen ein alternatives bildgebendes System, beispielsweise eine röntgenbasierte Durchleuchtung oder vorzugweise ein Ultraschallsystem, benötigt werden.

Die Ultraschalluntersuchung kann je nach Vorgabe mit unterschiedlichen Ultraschallköpfen durchgeführt werden. Von den dabei generierten Ultraschallmessdaten, welche im Wesentlichen den elektrischen Empfangssignalen entsprechen, können Ultraschallbilder ermittelt werden. Die ermittelten Ultraschallbilder können auf verschiedene Weise ausgewertet und dargestellt werden, was man als Mode (engl. für: Methode, Verfahren) bezeichnet. Neben dem A-Mode (Amplitudenverlauf der Ultraschallmessdaten) wird häufig ein zweidimensionales Schnittbild der zu untersuchenden Körperregion in Echtzeit erzeugt. Die Form des ermittelten Ultraschallbildes kann von dem Ultraschallkopf abhängig sein. Eine Zeitauflösung der Ultraschalluntersuchung ist üblicherweise eine Funktion von weiteren Messparametern der Ultraschalluntersuchung wie der Eindringtiefe der von dem Ultraschallkopf ausgeschickten Druckpulse und dem verwendeten Ultraschallkopf. Alternativ oder zusätzlich kann auch der B-Mode, M-Mode und/oder ein Doppler-Verfahren in einer Ultraschalluntersuchung auch in Kombination angewendet werden. Diese Verfahren sind allesamt dem Fachmann bekannt.

Die eine Vorgabe für die Ultraschalluntersuchung kann beispielsweise ein Ziel der Ultraschalluntersuchung aufweisen. Das Ziel der Ultraschalluntersuchung kann ein Erfassen einer Raumforderung in einem Organ während der Verlaufskontrolle sein. Üblicherweise wird bei der Verlaufskontrolle eine medizinische Untersuchung mehrmals, insbesondere über einen längeren Zeitraum hinweg durchgeführt. Auf Grund der üblicherweise risikoarmen, nicht-invasiven, schmerzlosen und strahlenexpositionsfreien Anwendung kann die mehrmalige Durchführung der medizinischen Untersuchung für den Patienten unbedenklich sein. Beispielsweise kann die Raumforderung, insbesondere ein Tumor oder ein Knoten, vor, während und nach der Therapie mit einem Medikament in einer Ultraschalluntersuchung erfasst werden. Die Vorgabe für die Ultraschalluntersuchung kann weiterhin das Erfassen des kardialen Blutflusses oder der Größe des Herzens sein.

In einer Ausführung weist die medizinische Fragestellung patientenspezifische Daten eines Patienten auf. Die patientenspezifische Daten weisen zumindest einen Parameter der folgenden Liste auf:

- eine Körpergröße des Patienten,
- ein Gewicht des Patienten,
- Befunde des Patienten und
- medizinische Bilddatensätze des Patienten.

Die patientenspezifischen Daten können derart zumindest eine anatomische Kennzahl des Patienten, wie beispielsweise die Körpergröße des Patienten und/oder das Gewicht des Patienten, umfassen. Der Bewegungsablauf des Roboterarms kann besonders geeignet auf die zumindest eine anatomische Kennzahl des Patienten abgestimmt sein. Beispielsweise kann der Bewegungsablauf räumlich kürzer sein, wenn der Patient schlanker ist. Als ein weiteres Beispiel kann der Bewegungsablauf räumlich länger sein, wenn der Patient größer ist.

Ferner können die patientenspezifischen Daten ein Alter des Patienten aufweisen. Das Alter des Patienten kann beispielsweise die Vorgabe der medizinischen Fragestellung beeinflussen. Die Vorgabe kann daher ein Kriterium sein, welches insbesondere die eigentliche Durchführung der Ultraschalluntersuchung spezifiziert oder charakterisiert.

Beispielsweise kann die Vorgabe eine zeitlich eher kurze Ultraschalluntersuchung anfordern. Die zeitlich eher kurze Ultraschalluntersuchung ist insbesondere vorteilhaft, wenn der zu untersuchende Patient nicht hinreichend lang ruhig liegen kann, weil der zu untersuchende Patient ein vergleichsweise hohes Alter hat. In anderen Worten, der Bewegungsablauf ist insbesondere desto zeitlich kürzer, je älter der Patient ist. Dann kann nämlich die Ultraschalluntersuchung schneller durchführbar sein. Üblicherweise werden manche Informationen über den Patienten, die beispielsweise bei Ultraschalluntersuchungen generiert werden, mit Bezug zu dem Patienten ausgewertet und gespeichert. Ein Nutzer, beispielsweise ein behandelnder Arzt des Patienten, beschreibt in einem Krankheitsbefund eine medizinische Verfassung des Patienten. Üblicherweise kann in dem Krankheitsbefund ein Hinweis auf eine Erkrankung (Art, Grad, etc.) sowie deskriptive Parameter stehen (Größe der Raumforderung, Blutdruck, etc.). Wenn der Krankheitsbefund bei einer nächsten medizinischen Untersuchung mit einer weiteren medizinischen Fragestellung vorliegt, kann sich der Arzt insbesondere mittels der Krankheitsbefunde über eine medizinische Historie des Patienten erkunden. Die medizinische Historie kann mehrere Krankheitsbefunde aufweisen, welche zu unterschiedlichen Gelegenheiten von verschiedenen Personen, insbesondere Ärzten erstellt wurden. Die patientenspezifischen Daten können daher beispielweise die Verlaufskontrolle der Tumorerkrankung oder einen Medikamentenplan enthalten. Beispielsweise kann gemäß der Verlaufskontrolle der Bewegungsdatensatz derart bestimmt werden, dass die Verlaufskontrolle durch die Ultraschalluntersuchung fortgeführt werden kann.

Für die medizinische Fragestellung können medizinische Bilddatensätze des Patienten relevant sein, welche vor der Ultraschalluntersuchung erfasst wurden. Beispielsweise können medizinische Bilddatensätze von unterschiedlichen medizinischen Bildgebungsgeräten erfasst worden sein. Außerdem können medizinische Bilddatensätze auch übliche Kameraaufnahmen von der Körperoberfläche des Patienten aufweisen. Unter Berücksichtigung der medizinischen Bilddatensätze kann vorzugweise ein Bewegungsdatensatz derart bestimmt werden, dass die Ultraschalluntersuchung mit hoher Qualität durchgeführt werden kann.

Aus Gründen der Übersichtlichkeit werden die Ausführungen, welche zumindest eine medizinische Trainingsfragestellung betreffen, nicht separat aufgeführt, weil diese im Wesentlichen den Ausführungen über die medizinische Fragestellung entsprechen. Das bedeutet im Wesentlichen, dass sich die medizinische Trainingsfragestellung und die medizinische Fragestellung bis auf den Zeitpunkt des jeweiligen Erfassens in ihrem Datenformat entsprechen können. Der Roboterarm weist üblicherweise geeignete Befestigungsmittel dafür auf, dass der Ultraschallkopf über die Befestigungsmittel an dem Roboterarm befestigt werden kann.

Der Roboterarm ist vorzugweise derart ausgestaltet, dass dieser die üblichen Bewegungen eines menschlichen Nutzers ausführen kann. Beispielsweise kann der Roboterarm dafür zumindest ein Drehgelenk und zumindest ein Trägerelement aufweisen. Es ist auch denkbar, dass der Roboterarm zumindest eine Teleskopeinheit, welche eingefahren und ausgefahren werden kann, aufweist. Beispielsweise kann an dem zumindest einen Trägerelement der Ultraschallkopf befestigt sein. Bei geeigneter Konfiguration des Roboterarms weist der Roboterarm zumindest einen Freiheitsgrad auf. Üblicherweise weist der Roboterarm so viele Freiheitsgrade auf, dass der Roboterarm eine Aufgabe, welche dem Roboterarm gestellt worden ist, ausführen kann. Die Ausführung der dem Roboterarm gestellten Aufgabe umfasst üblicherweise das Bewegen des Roboterarms gemäß einem Bewegungsdatensatz.

Der Roboterarm weist normalerweise ein Roboterarm-Koordinatensystem auf, welches einen Bewegungsfreiraum des Roboterarms enthält, in welchem der Roboterarm bewegt werden kann. Der durch das trainierte neuronale Netz bestimmte Bewegungsdatensatz ist vorzugweise auf den Bewegungsfreiraum des Roboterarms abgestimmt. Der Bewegungsfreiraum des Roboterarms ist insbesondere durch die Ausgestaltung des Roboterarms in Hinblick auf vorgesehene Freiheitsgrade des Roboterarms vorgegeben. Der Bewegungsfreiraum kann beispielsweise mittels einer Kalibrierungsfahrt erfasst worden sein. Je nach Art und Konfiguration des Roboterarms kann sich der Bewegungsfreiraum des Roboterarms unterscheiden.

Das neuronale Netz kann abhängig von der Eingabe an der Eingangsschicht eine Ausgabe an der Ausgangsschicht bereitstellen. Üblicherweise wird an der Eingangsschicht des bereitgestellten trainierten künstlichen neuronalen Netzes die erfasste medizinische Fragestellung eingegeben. Die medizinische Fragestellung kann als Eingabeinformation in das trainierte künstliche neuronale Netz eingegeben werden. Das künstliche neuronale Netz kann als Ausgabe, insbesondere als Ausgabe der künstlichen Neuronen der Ausgabeschicht, den Bewegungsdatensatz bestimmen. In anderen Worten wird der Bewegungsdatensatz als Ausgabe der künstlichen Neuronen der Ausgabeschicht zu der medizinischen Fragestellung zugeordnet. Das Bereitstellen der Ausgabe an der Ausgangsschicht nach Eingabe an der Eingangsschicht kann einem Zuordnen des Bewegungsdatensatzes abhängig von der medizinischen Fragestellung entsprechen, wenn das künstliche neuronale Netz derart auf die medizinische Fragestellung angewendet wird, dass die medizinische Fragestellung als Eingabeinformation an der Eingangsschicht des künstlichen neuronalen Netzes eingegeben wird und darauf basierend ein Bewegungsdatensatz bestimmt wird.

Diesem Vorgehen liegt insbesondere die Überlegung zugrunde, dass der Bewegungsdatensatz über die medizinische Fragestellung ausgelesen werden kann. So wie für einen menschlichen Nutzer, insbesondere einen Arzt, allein anhand der medizinischen Fragestellung feststellbar ist, mit welchem Bewegungsdatensatz die Ultraschalluntersuchung durchführbar ist, so kann das entsprechend trainierte künstliche neuronale Netz diese Informationen ebenfalls allein auf Grundlage der medizinischen Fragestellung extrahieren.

Der durch die Anwendung des künstlichen neuronalen Netzes bestimmte Bewegungsdatensatz wird an die Steuereinheit des Roboterarms übertragen. Der Bewegungsdatensatz kann in einem geeigneten Datenformat vorliegen, welches von der Ausgabeschicht des neuronalen Netzes direkt oder indirekt an die Steuereinheit des Roboterarms übertragen wird. Die Steuereinheit des Roboterarms kann üblicherweise den Bewegungsdatensatz geeignet verarbeiten, sobald der Bewegungsdatensatz übertragen ist.

Der Bewegungsdatensatz weist den Bewegungsablauf des Roboterarms auf. Darüber hinaus kann der Bewegungsdatensatz eine Konfiguration darüber enthalten, inwiefern der Bewegungsablauf eine räumliche Toleranz bei einer Abweichung vom vorgegebenen Bewegungsablauf aufweist.

In einer weiteren Ausgestaltung umfasst der Bewegungsablauf des Roboterarms die Funktion der Orientierung des Roboterarms über die Zeit und die Funktion der Position des Roboterarms über die Zeit. Der Bewegungsablauf kann daher insbesondere das Bewegen des Roboterarms beschreiben. Die Position des Roboterarms, insbesondere eines Mittelpunkts oder eines Endpunkts des Roboterarms, kann üblicherweise durch Koordinaten in einem dreidimensionalen Koordinatensystem mit einem beliebigen Bezugspunkt festgelegt sein. Die Orientierung des Roboterarms kann insbesondere einem Raumwinkel zwischen beispielsweise einer sich in Längsrichtung des Roboterarms erstreckenden Roboterachse und einer Systemebene, welche durch das Koordinatensystem definiert ist, spezifiziert sein.

Eine Kombination von Position und Orientierung wird im technischen Zusammenhang häufig als Pose oder räumliche Lage bezeichnet. Die Funktion der räumlichen Lage über die Zeit entspricht daher insbesondere der Funktion der Orientierung über die Zeit zusammen mit der Funktion der Position über die Zeit. Der Bewegungsablauf kann daher zumindest eine räumliche Lage über die Zeit aufweisen. Beispielsweise kann eine Robotersteuerung über eine Schnittstelle Bewegungsabläufe empfangen oder senden. Die Bewegungsabläufe des Roboterarms können unmittelbar in die Funktion der Orientierung des Roboterarms über die Zeit und die Funktion der Position des Roboterarms über die Zeit übersetzt werden.

Die Steuereinheit kann gemäß dem Bewegungsablauf des Bewegungsdatensatzes den Roboterarm bewegen, beispielsweise indem die Steuereinheit Steuersignale, die dem Bewegungsablauf entsprechen, an den Roboterarm sendet. Insbesondere kann sich der Roboterarm entlang der räumlichen Lagen, die dem Bewegungsablauf entsprechen, bewegen. Der Roboterarm fährt daher vorzugweise die räumliche Lagen, insbesondere die von der zeitlichen Funktion angegebenen Positionen und Orientierungen, an. Demnach steuert vorzugweise die Steuereinheit das Bewegen des Roboterarms gemäß dem Bewegungsablauf des Bewegungsdatensatzes. Vorzugweise wird der Roboterarm auf der Körperoberfläche des Patienten bewegt. Wenn die medizinische Fragestellung eine bestimmte Körperregion aufweist, wird der Roboterarm gemäß der bestimmten Körperregion bewegt.

Aus Gründen der Übersichtlichkeit werden die Ausführungen, welche den zumindest einen Trainingsbewegungsdatensatz betreffen, nicht separat aufgeführt, weil diese im Wesentlichen den Ausführungen über den Bewegungsdatensatz entsprechen. Analog entspricht ein Trainingsbewegungsablauf im Wesentlichen dem Bewegungsablauf.

In einer weiteren Ausführung weist der Roboterarm Befestigungsmittel für einen Projektor auf. Vorzugweise ist der Projektor mittels der Befestigungsmittel mit dem Roboterarm lösbar verbunden. Grundsätzlich ist auch denkbar, dass der Roboterarm einen externen Projektor, der nicht an dem Roboterarm befestigt ist, beispielsweise über eine Kabelverbindung ansteuert. Beispielsweise kann der Projektor mittels LaserLicht den Bewegungsablauf auf die Körperoberfläche des Patienten projizieren.

Grundsätzlich ist es auch denkbar, dass der Projektor den Nutzer beim Erfassen der medizinischen Fragestellung unterstützt. Beispielsweise kann der Projektor die zu untersuchende Körperregion des Patienten mit Licht kennzeichnen oder erhellen.

Gemäß einer weiteren Ausführung weist der Roboterarm einen Sensor auf. Alternativ ist es auch denkbar, dass der Roboterarm Befestigungsmittel für den Sensor aufweist, wobei der Sensor mittels der Befestigungsmittel an dem Roboterarm lösbar befestigt werden kann. Der Sensor weist zumindest eine der folgenden Varianten auf: einen elektro-optischen Sensor, eine Kamera, eine Vorrichtung zum Erfassen eines Elektrokardiogramms, einen Abstandssensor, einen Lagesensor und einen Drucksensor. Der Sensor kann die räumliche Lage des Roboterarms erfassen. Der Sensor kann üblicherweise erfassen, ob der Bewegungsablauf des Roboterarms durch ein Hindernis beeinträchtigt ist. Der Sensor bietet normalerweise eine Möglichkeit der Rückkopplung beim Bewegen des Roboterarms.

Der Lagesensor kann den Bewegungsablauf des Roboterarms, insbesondere die Funktion der Orientierung des Roboterarms über die Zeit und die Funktion der Position des Roboterarms über die Zeit, erfassen. Alternativ oder zusätzlich kann der Sensor einen Beschleunigungssensor aufweisen. Grundsätzlich ist es denkbar, dass der Roboterarm mehrere Sensoren aufweist. Beispielsweise kann der Roboterarm einen ersten Lagesensor für ein Robotergelenk, welches dem zumindest einen Drehgelenk des Roboterarms entsprechen kann, und einen zweiten Lagesensor sowie einen Beschleunigungssensor im Ultraschallkopf aufweisen.

Beispielsweise kann der Drucksensor ermitteln, ob der Roboterarm sich frei bewegt oder beispielsweise zu fest gegen den Patienten gedrückt wird. Beispielsweise kann der Roboterarm eine geeignete Vorrichtung, beispielsweise einen Kollisionssensor oder einen Algorithmus aufweisen, der eine Kollision des Roboterarms mittels des Sensors mit dem Patienten detektieren kann. Beispielsweise kann eine Atembewegung des Patienten erfasst werden. Die Atembewegung des Patienten wird beispielsweise mit einem Atemgurt zur Erfassung der Atembewegung oder mit der Kamera erfasst.

Die Steuereinheit kann mittels des Sensors den Bewegungsablauf erfassen. Alternativ oder zusätzlich kann die Steuereinheit den Bewegungsablauf mittels geeigneter Algorithmen berechnen und erfassen. Die Steuereinheit des Roboterarms verfügt insbesondere über geeignete Algorithmen, die die räumliche Lage des Roboterarms erfassen, insbesondere berechnen, können.

Normalerweise validiert die Steuereinheit den bestimmten Bewegungsdatensatz in Hinblick auf Durchführbarkeit. Dabei kann die Steuereinheit auf weitere Informationen des Sensors zurückgreifen. Der Bewegungsdatensatz kann durch die Steuereinheit auf den Bewegungsfreiraum des Roboterarms angepasst werden. Falls keine Anpassung möglich ist, kann die Steuereinheit beispielsweise die Ultraschalluntersuchung unterbrechen und/oder eine Fehlermeldung zurückgeben. Der Bewegungsfreiraum des Roboterarms weist üblicherweise den Bewegungsfreiraum des Ultraschallkopfes auf, der an dem Roboterarm befestigt ist. Innerhalb des Bewegungsfreiraums des Ultraschallkopfes kann der Ultraschallkopf bewegt werden.

Die Validierung des Bewegungsdatensatzes durch die Steuereinheit des Roboterarms kann umfassen, dass die Steuereinheit mittels der von dem Sensor bereitgestellten Daten, von welchen beispielsweise Bilder rekonstruiert werden, den bestimmten Bewegungsdatensatz derart korrigiert, dass der Bewegungsdatensatz an die Körperoberfläche des Patienten angepasst wird. Dies kann insbesondere einer besseren Durchführung der Ultraschalluntersuchung dienen, wenn beispielsweise seit dem Trainieren des künstlichen neuronalen Netzes der Patient beispielsweise älter oder kränker oder schlanker geworden ist. Für die Validierung des Bewegungsdatensatzes kann relevant sein, dass Ultraschallbilder von der Ultraschalluntersuchung generiert werden können, die für die medizinische Fragestellung geeignet sind.

Insbesondere die Steuereinheit des Roboterarms sendet geeignete Steuersignale aus, damit der Roboterarm bewegt wird. Wenn der Roboterarm bewegt wird, kann üblicherweise der Roboterarm durch Motoren und/oder Aktoren und/oder Sensoren des Roboterarms bewegt werden.

In einer weiteren Ausführungsform wird der Roboterarm gemäß dem Bewegungsablauf des Bewegungsdatensatzes automatisch bewegt. Es ist daher denkbar, dass in einem Untersuchungsraum sich lediglich der Roboterarm mit dem Patienten befindet. Eine Kontrollperson kann sich in dem Untersuchungsraum aufhalten. Es wäre auch denkbar, dass die Kontrollperson das automatische Bewegen des Roboterarms freigibt. Beispielsweise wird der Roboterarm gemäß des Bewegungsablaufs lediglich bewegt, während die Kontrollperson, insbesondere durch das Betätigen einer Sicherungsvorrichtung, das Bewegen des Roboterarms freigibt. Sobald die Kontrollperson das Freigeben des Bewegens unterbricht, wird insbesondere das Bewegen gemäß dem Bewegungsablauf ebenfalls unterbrochen. Normalerweise wird nach einer Unterbrechung der Freigabe durch die Kontrollperson das Bewegen des Roboterarms gemäß dem Bewegungsablauf wieder aufgenommen.

Die Sicherungsvorrichtung kann alternativ oder zusätzlich durch die Kontrollperson außerhalb des Untersuchungsraums betätigt werden. Vorzugweise kann die Kontrollperson sich an einem beliebigen Ort befinden, wobei die Kontrollperson beispielsweise über eine geeignete Netzwerk-Verbindung und eine entsprechend eingerichtete Anzeigevorrichtung verfügt, welche eine Kontrolle der Ultraschalluntersuchung von außerhalb des Untersuchungsraum ermöglichen. Beispielsweise ist es auch denkbar, dass die Kontrollperson eine Intervention oder einen chirurgischen Eingriff bei dem Patienten durchführt. In diesem Fall kann der Roboterarm automatisch verfahren werden, während beispielsweise die Kontrollperson, insbesondere der Arzt, die Intervention oder den chirurgischen Eingriff durchführt.

Gemäß einer weiteren Ausführung wird die Abweichung einer Bewegung des Roboterarms von dem Bewegungsablauf des Bewegungsdatensatzes beim Bewegen des Roboterarms mittels des Sensors ermittelt. Die Abweichung von dem Bewegungsablauf kann mittels eines geeigneten Sensors insbesondere in Echtzeit erfasst werden. Eine tatsächliche räumliche Lage, insbesondere ein tatsächlicher Bewegungsablauf, des Roboterarms kann von der durch den Bewegungsablauf vorgegeben räumliche Lage zu einem beliebigen Zeitpunkt abweichen, beispielsweise weil der Patient atmet.

Die Steuereinheit weist insbesondere Mittel für das Gegensteuern auf, wenn das Bewegen des Roboterarms von dem Bewegungsablauf des Bewegungsdatensatzes abweicht. Beispielsweise kann die Steuereinheit gemäß der ermittelten Abweichung, beispielsweise gemäß der Atembewegung des Patienten den Bewegungsablauf des Roboterarms anpassen und gegensteuern. Die Steuereinheit kann üblicherweise Korrektur-Steuersignale gemäß der Vorrichtung zum Erfassen des Echokardiogramms berechnen. Das Gegensteuern kann bedeuten, dass die Steuereinheit Korrektur-Steuersignale berechnet und die Korrektur-Steuersignale an den Roboterarm sendet, wobei der Roboterarm gemäß den Korrektur-Steuersignalen bewegt wird. Die Steuereinheit kann normalerweise unabhängig von dem Grund, warum die Abweichung von dem Bewegungsablauf entstanden ist, geeignete Korrektur-Steuersignale berechnen und an den Roboterarm senden.

Gemäß einer weiteren Ausführung erfolgt das Bewegen des Roboterarms gemäß dem Bewegungsablauf des Bewegungsdatensatzes durch den Nutzer. Beispielsweise kann der Nutzer den Roboterarms bewegen, wenn der Nutzer den Ultraschallkopf mit zumindest einer seiner Hände während der Ultraschalluntersuchung führt. Der Roboterarm kann eine geeignete Führungsvorrichtung vorweisen, welche der Nutzer in zumindest eine Hand nimmt oder mit zumindest einer Hand führen kann. Das Verfahren des Roboterarms kann einem Führen des Ultraschallkopfs durch den Nutzer entsprechen. Das Führen des Ultraschallkopfs kann derart ausgeführt sein, dass der Nutzer den Ultraschallkopf mit zumindest einer Hand führen kann oder der Nutzer die geeignete Führungsvorrichtung mit zumindest einer Hand führt, wobei über eine direkte Wirkverbindung zwischen der geeignete Führungsvorrichtung und dem Roboterarm der Ultraschallkopf verfahren wird. Beispielsweise kann der Nutzer entweder direkt den Ultraschallkopf führen oder mittels der geeigneten Führungsvorrichtung den Ultraschallkopf führen. Die direkte Wirkverbindung kann Mittel dafür aufweisen, dass der Roboterarm und/oder der an dem Roboterarm befestigte Ultraschallkopf verfahren werden, wenn die geeignete Führungsvorrichtung verfahren wird. Die direkte Wirkverbindung kann beispielsweise einen Hebel aufweisen.

Das Bewegen des Roboterarms gemäß dem Bewegungsablauf des Bewegungsdatensatzes durch den Nutzer umfasst das Unterstützen des Nutzers beim Bewegen des Roboterarms gemäß dem Bewegungsablauf des Bewegungsdatensatzes, beispielsweise wenn ein Eigengewicht des Roboterarms durch die Steuereinheit kompensiert wird. Vorzugweise kann der Nutzer die Ultraschalluntersuchung ohne Beeinträchtigung durch den Roboterarm, an welchem der Ultraschallkopf befestigt ist, durchführen. Die Beeinträchtigung durch den Roboterarm kann auf Grund eines fehlenden Freiheitsgrades des Roboterarms erfolgen. Alternativ oder zusätzlich kann die Beeinträchtigung einen Widerstand beim Bewegen des Roboterarms, welcher üblicherweise durch die Reibung beispielsweise in einem Drehgelenk oder beim Einfahren und Ausfahren einer Teleskopeinheit entstehen kann, aufweisen. Vorzugweise ist der den Ultraschallkopf führende Nutzer beim Bewegen des Roboterarms, insbesondere beim Führen des Ultraschallkopfes frei. In anderen Worten, der Nutzer wird derart unterstützt, dass der Nutzer ohne zusätzliche Kraftanstrengung den Roboterarm gemäß dem Bewegungsablauf des Bewegungsdatensatzes bewegen kann, als ob der Ultraschallkopf nicht an dem Roboterarm befestigt wäre.

Wenn ein Bewegungsdatensatz durch Anwendung des künstlichen neuronalen Netzes auf die medizinische Fragestellung bestimmt ist und wenn das Bewegen des Roboterarms von dem Bewegungsablauf des Bewegungsdatensatzes abweicht, kann beispielsweise der Nutzer derart unterstützt werden, dass die Steuereinheit des Roboterarms den Nutzer darauf aufmerksam macht. Beispielsweise kann der Roboterarm eine Vibrationsvorrichtung aufweisen, die mittels einer Vibration den Nutzer darauf aufmerksam macht, wenn das Bewegen des Roboterarms von dem Bewegungsablauf des Bewegungsdatensatzes abweicht. Alternativ oder zusätzlich wäre auch denkbar, dass der Roboterarm mittels einer optischen Vorrichtung, insbesondere einer Lampe oder einem Laser, oder mittels des Projektors dem Nutzer den Bewegungsablauf des bestimmten Bewegungsdatensatzes anzeigt. Die Lampe könnte beispielsweise bei einer Abweichung von dem Bewegungsablauf des bestimmten Bewegungsdatensatzes aufleuchten. Alternativ oder zusätzlich kann der Laser den Bewegungsablauf des bestimmten Bewegungsdatensatzes insbesondere auf die Körperoberfläche projizieren.

In einer weiteren Ausführung ist es denkbar, dass der Roboterarm gemäß dem Bewegungsablauf des Bewegungsdatensatzes sowohl automatisch als auch durch den Nutzer bewegt werden kann. In diesem Fall kann der Roboterarm sowohl die geeignete Führungsvorrichtung aufweisen als auch beispielsweise die Sicherungsvorrichtung, welche üblicherweise der Roboterarm aufweist, wenn der Roboterarm automatisch bewegt werden kann. Beispielsweise abhängig von der Ultraschalluntersuchung kann entweder der Nutzer den Roboterarm gemäß dem Bewegungsablauf des Bewegungsdatensatzes bewegen oder der Roboterarm wird automatisch gemäß dem Bewegungsablauf des Bewegungsdatensatzes bewegt.

Weiterhin erfolgt das Bereitstellen des trainierten künstlichen neuronalen Netzes gemäß dem Verfahren zu einem Bereitstellen eines trainierten künstlichen neuronalen Netzes für ein Bestimmen eines Bewegungsdatensatzes, welcher einen Bewegungsablauf eines Roboterarms für eine Ultraschalluntersuchung aufweist, wobei an dem Roboterarm ein Ultraschallkopf befestigt ist. Derart kann ein besonders vorteilhaft trainiertes künstliches neuronales Netz für die Aufgabe des Bestimmens eines Bewegungsdatensatzes durch Anwendung des trainierten künstlichen neuronalen Netzes auf die medizinische Fragestellung bereitgestellt werden.

Das Verfahren zu einem Bereitstellen eines trainierten künstlichen neuronalen Netzes für ein Bestimmen eines Bewegungsdatensatzes, welcher einen Bewegungsablauf eines Roboterarms für eine Ultraschalluntersuchung aufweist, wobei an dem Roboterarm ein Ultraschallkopf befestigt ist, , umfasst folgende Verfahrensschritte:

- Bereitstellen zumindest eines Trainingsbewegungsdatensatzes, wobei dem zumindest einen Trainingsbewegungsdatensatz zumindest eine medizinische Trainingsfragestellung zugeordnet ist,
- Training eines künstlichen neuronalen Netzes unter Verwendung des zumindest einen Trainingsbewegungsdatensatzes und der zumindest einen medizinischen Trainingsfragestellung, wobei die Anwendung des trainierten künstlichen neuronalen Netzes auf die zumindest eine medizinische Trainingsfragestellung ein Bestimmen des zumindest einen Trainingsbewegungsdatensatzes ermöglicht und
- Bereitstellen des trainierten künstlichen neuronalen Netzes für ein Bestimmen des Bewegungsdatensatzes.

Für das Training des künstlichen neuronalen Netzes ist also der zumindest eine Trainingsbewegungsdatensatz und die zumindest eine medizinische Trainingsfragestellung, wobei dem zumindest einen Trainingsbewegungsdatensatz zumindest eine medizinische Trainingsfragestellung zugeordnet ist, entscheidend.

Insbesondere ist die zumindest eine medizinische Trainingsfragestellung bereits dem zumindest einen Trainingsbewegungsdatensatz zugeordnet. Ansonsten kann das Zuordnen der zumindest einen medizinischen Trainingsfragestellung zu dem zumindest einen Trainingsbewegungsdatensatz manuell oder automatisch erfolgen. Das Zuordnen der zumindest einen medizinischen Trainingsfragestellung zu dem zumindest einen Trainingsbewegungsdatensatz kann dabei beispielsweise von einem Hersteller des Sensors, mit welchem die Bewegungsdatensätze erfasst worden sind, und/oder von einem Nutzer, der die Bewegungsdatensätze basierend auf Untersuchungen generiert hat, oder von einem Personal in einer Klinik durchgeführt werden.

Üblicherweise liegen zu dem zumindest einem Trainingsbewegungsdatensatz die zumindest eine medizinische Trainingsfragestellung, insbesondere patientenspezifische Daten vor. Beispielsweise kann ein Bildformat (z.B. DICOM) zusätzlich zu der Bildinformation patientenspezifische Daten, insbesondere medizinische Fragestellungen aufweisen. Insbesondere wird nur der zumindest eine Trainingsbewegungsdatensatz für das Training des künstlichen neuronalen Netzes verwendet, welchem die zumindest eine medizinische Trainingsfragestellung zugeordnet ist, wobei die zumindest eine medizinische Trainingsfragestellung ausreichende Informationen für das Training aufweisen.

Das Erfassen des zumindest einen Trainingsbewegungsdatensatzes und das Bereitstellen des zumindest einen Trainingsbewegungsdatensatzes kann vorzugweise mittels des Sensors, beispielsweise des Lagesensors und/oder des Beschleunigungssensors, erfolgen. Das Erfassen des zumindest einen Trainingsbewegungsdatensatzes geht üblicherweise dem Bereitstellen des zumindest einen Trainingsbewegungsdatensatzes voraus. Beispielsweise erfasst der Sensor zunächst den zumindest eine Trainingsbewegungsdatensatz, in dem der Bewegungsablauf erfasst wird, und in einem nächsten Schritt stellt insbesondere eine Steuereinheit des Sensors über eine geeignete Schnittstelle den Trainingsbewegungsdatensatz bereit.

Gemäß einer Ausführungsform weist das Bereitstellen des zumindest einen Trainingsbewegungsdatensatz durch den Nutzer das manuelle Durchführen der zumindest einen Trainingsultraschalluntersuchung mittels des Roboterarms basierend auf der zumindest einen medizinischen Fragestellung und das Erfassen des Trainingsbewegungsablauf des zumindest einen Trainingsbewegungsdatensatzes während der zumindest einen manuell durchgeführten Trainingsultraschalluntersuchung auf.

Das manuelle Durchführen der zumindest einen Trainingsultraschalluntersuchung mittels des Roboterarms erfolgt üblicherweise durch den Nutzer. Beispielsweise kann der Nutzer mittels der Führungsvorrichtung des Roboterarms den Ultraschallkopf bewegen. Üblicherweise basiert die zumindest eine Trainingsultraschalluntersuchung auf der zumindest einen medizinischen Trainingsfragestellung.

Beim Bewegen des Roboterarms kann der Trainingsbewegungsablauf, insbesondere die über die Zeit abgefahrenen räumlichen Lagen gespeichert werden. Bei der manuellen Durchführung der zumindest einen Trainingsultraschalluntersuchung kann mittels des Roboterarms, insbesondere der Steuereinheit des Roboterarms basierend auf der zumindest einen medizinischen Trainingsfragestellung der Trainingsbewegungsablauf erfasst werden. Notwendigerweise kann der in diesem Fall basierend auf der zumindest einen medizinischen Trainingsfragestellung erfasste Trainingsbewegungsablauf in den zumindest einen Trainingsbewegungsdatensatz übersetzt werden. Der zumindest eine Trainingsbewegungsdatensatz weist dann vorzugweise den Trainingsbewegungsablauf auf, gemäß welchem der Roboterarm durch den Nutzer bei der manuellen Durchführung von der zumindest einen Trainingsultraschalluntersuchung basierend auf der zumindest einen medizinischen Trainingsfragestellung bewegt wurde. Daher kann bei der manuellen Durchführung von der zumindest einen Trainingsultraschalluntersuchung die zumindest eine medizinische Trainingsfragestellung zu dem zumindest einen Trainingsbewegungsdatensatz zugeordnet werden.

Alternativ oder zusätzlich kann gemäß einer weiteren Ausführungsform das Bereitstellen des zumindest einen Trainingsbewegungsdatensatzes durch den Nutzer das ausschließlich manuelle Durchführen zumindest einer Trainingsultraschalluntersuchung mittels eines freien Ultraschallkopfes, welcher nicht an dem Roboterarm befestigt ist, basierend auf der zumindest einen medizinischen Trainingsfragestellung, wobei der Nutzer den freien Ultraschallkopf bewegt, das Erfassen der Bewegungen des freien Ultraschallkopfs während der zumindest einen Trainingsultraschalluntersuchung mittels eines Sensors und das Übersetzen der erfassten Bewegungen in zumindest einen mittels des Roboterarms durchführbaren Trainingsbewegungsdatensatz aufweisen.

Die zumindest eine Trainingsultraschalluntersuchung kann beispielsweise ohne den Ultraschallkopf, welcher mit dem Roboterarm befestigt ist, durchgeführt werden. Beispielsweise kann der freie Ultraschallkopf auch dem an dem Roboterarm befestigten Ultraschallkopf entsprechen, wenn der andernfalls freie Ultraschallkopf am Roboterarm befestigt ist. Grundsätzlich kann der freie Ultraschallkopf auch ein zweiter Ultraschallkopf neben dem am Roboterarm befestigten Ultraschallkopf sein. Weiterhin ist es auch möglich, dass sich lediglich der freie Ultraschallkopf mit einem zugehörigen Ultraschallsystem in einem Trainingsraum zur Verfügung steht und der Roboterarm in dem Untersuchungsraum befindet.

Die Bewegungen des freien Ultraschallkopfs während der zumindest einen Trainingsultraschalluntersuchung werden mittels eines Sensors, beispielsweise einer Kamera erfasst. Das Erfassen der Bewegungen kann dem Speichern von Bewegungsdaten entsprechen. Die Bewegungsdaten entsprechen üblicherweise den erfassten Bewegungen, insbesondere der räumlichen Lage des freien Ultraschallkopfs über die Zeit während der zumindest einen Trainingsultraschalluntersuchung. Gemäß der räumlichen Lage des freien Ultraschallkopfs über die Zeit kann der Bewegungsablauf ermittelt werden, welcher in den zumindest einen mittels des Roboterarms durchführbaren Trainingsbewegungsdatensatz übersetzt wird. Das Übersetzen der erfassten Bewegungsdaten, insbesondere der erfassten Bewegungen entspricht üblicherweise einem Umrechnen der Bewegungsdaten, insbesondere der Bewegungen in den zumindest einen mittels des Roboterarms Trainingsbewegungsdatensatz. Bei der Umrechnung wird vorzugweise der Bewegungsfreiraum des Roboterarms berücksichtigt.

Das Training des künstlichen neuronalen Netzes erfolgt vorteilhafterweise mittels Rückwärtspropagation (Backpropagation). Dieses bedeutet insbesondere, dass die zumindest eine Trainingsfragestellung als Eingangsdaten in das zu trainierende künstliche neuronale Netz eingespeist werden. Während des Trainings wird dann eine Ausgabe des zu trainierenden künstlichen neuronalen Netzes mit dem zumindest einen Trainingsdatensatz verglichen, der der zumindest einen Trainingsfragestellung zugeordnet ist. Die Ausgabe des zu trainierenden künstlichen neuronalen Netzes weist vorzugweise den zumindest einen Trainingsbewegungsdatensatz auf. Das Training des künstlichen neuronalen Netzes umfasst dann insbesondere eine Veränderung der Netzwerkparameter des zu trainierenden künstlichen neuronalen Netzes derart, dass die Ausgabe des zu trainierenden künstlichen neuronalen Netzes näher an dem zumindest einen Trainingsdatensatz liegt, dem die zumindest eine Trainingsfragestellung zugeordnet ist. Daher wird das künstliche neuronale Netz vorteilhafterweise derart trainiert, dass durch die Anwendung des künstlichen neuronalen Netzes auf die zumindest eine Trainingsfragestellung der der zumindest einen Trainingsfragestellung zugeordnete zumindest eine Trainingsbewegungsdatensatz bestimmt wird.

Während die Rückwärtspropagation normalerweise den wichtigsten Trainingsalgorithmus zum Training des künstlichen neuronalen Netzes darstellt, können auch andere, dem Fachmann bekannte Algorithmen zum Training des künstlichen neuronalen Netzes eingesetzt werden. Beispiele für andere mögliche Algorithmen sind evolutionäre Algorithmen, "simulated annealing", "Expectaction-Maximization" Algorithmen (EM-Algorithmen), parameterfreie Algorithmen (non-parametric methods), Partikelschwarmoptimierung (PSO), usw.

Das Training des künstlichen neuronalen Netzes kann komplett bei dem Hersteller des Roboterarms und/oder bei dem Unternehmen stattfinden, das das Training durchführt. Alternativ ist es auch denkbar, dass ein Vor-Training bei dem Hersteller des Roboterarms und/oder bei dem Unternehmen stattfinden, das das Training initial durchführt, angelegt wird und ein Nach-Training einmalig oder mehrfach in einer Klinik angelegt wird, um das Bestimmen des Bewegungsdatensatzes spezifisch für die Anforderungen der Klinik noch robuster zu gestalten. Ebenso ist es denkbar, ein bereits trainiertes künstliches neuronales Netz durch das Einspielen neuer Gewichtsmatrizen für eine andere Klassifizierungsaufgabe umzuwidmen.

Es ist auch denkbar, dass das Training des künstlichen neuronalen Netzes in mehreren Iterationen erfolgt. Derart können in mehreren Schritten abwechselnd ein Bereitstellen des zumindest einen Trainingsbewegungsdatensatzes, wobei dem zumindest einen Trainingsbewegungsdatensatz zumindest eine medizinische Trainingsfragestellung zugeordnet ist, und das Training des künstlichen neuronalen Netzes erfolgen. So kann beispielsweise eine Trennschärfe bei dem Bestimmen des Bewegungsdatensatzes mittels des trainierten künstlichen neuronalen Netzes verbessert werden.

Das derart trainierte künstliche neuronale Netz kann anschließend in einem erfindungsgemäßen Verfahren zum Bestimmen eines Bewegungsdatensatzes durch Anwendung des trainierten künstlichen neuronalen Netzes auf eine medizinische Fragestellung eingesetzt oder dafür bereitgestellt werden. Derart ermöglicht das beschriebene Training des künstlichen neuronalen Netzes ein anschließend besonders vorteilhaftes Bestimmen von Bewegungsdatensätzen, welche noch nicht im Voraus bekannt sind, abhängig von medizinischen Fragestellungen.

Üblicherweise kann das Verfahren zu einem Bereitstellen eines trainierten künstlichen neuronalen Netzes vorsehen, dass vor dem Bereitstellen des trainierten künstlichen neuronalen Netzes eine Überprüfung einer Validität des trainierten künstlichen neuronalen Netzes erfolgt. Die Überprüfung der Validität kann lediglich mit einer Stichprobe erfolgen. Mit dieser Überprüfung kann sichergestellt werden, dass das trainierte künstliche neuronale Netz für das Bestimmen eines Bewegungsdatensatzes geeignet ist.

Das Ultraschallsystem umfasst eine Planungseinheit, eine erste Recheneinheit und eine Messeinheit, welche einen Roboterarm für eine Ultraschalluntersuchung, wobei an dem Roboterarm ein Ultraschallkopf befestigt ist, aufweist.

Die Planungseinheit kann den Monitor mit der graphischen Benutzeroberfläche und das Eingabegerät aufweisen. Üblicherweise kann der Nutzer mit der Planungseinheit, insbesondere durch das Eingabegerät interagieren. Beispielsweise kann der Nutzer auf dem Monitor auch Ultraschallmessdaten oder Ultraschallbilder der Ultraschalluntersuchung wahrnehmen. Die Planungseinheit kann Ultraschallmessdaten insbesondere auf dem Monitor anzeigen.

Die erste Recheneinheit ist vorzugweise derart konfiguriert, dass die erste Recheneinheit das Bestimmen des Bewegungsdatensatzes durch Anwendung des künstlichen neuronalen Netzes auf die medizinische Fragestellung durchführen kann. Die Recheneinheit kann Schnittstellen zur Planungseinheit, zur Steuereinheit oder zur Messeinheit aufweisen, wobei über die Schnittstellen die medizinische Fragestellung und/oder der Bewegungsdatensatz insbesondere empfangen und versendet werden können. Die erste Recheneinheit ist vorzugweise derart ausgestaltet, dass das trainierte neuronale Netz, welches beispielsweise als ein Computerprogrammprodukt bereitgestellt ist und in einen Speicher der ersten programmierbaren Recheneinheit geladen werden kann, auf der ersten Recheneinheit ausführbar ist.

Die Messeinheit weist den Roboterarm für die Ultraschalluntersuchung auf. Der Ultraschallkopf ist an dem Roboterarm befestigt. Die Messeinheit umfasst normalerweise den Roboterarm und die Steuereinheit des Roboterarms. Die Messeinheit kann den Sensor, die Kamera und/oder den Projektor aufweisen. Das Ultraschallsystem ist vorzugsweise zum Bewegen des Roboterarms für die Ultraschalluntersuchung ausgebildet.

Die Komponenten des erfindungsgemäßen Ultraschallsystems können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die zweite Recheneinheit ist zu einem Bereitstellen eines trainierten künstlichen neuronalen Netzes für ein Bestimmen eines Bewegungsdatensatzes, welcher einen Bewegungsablauf eines Roboterarms für eine Ultraschalluntersuchung, wobei an dem Roboterarm ein Ultraschallkopf befestigt ist, aufweist, ausgebildet. Vorzugweise kann die zweite Recheneinheit ein Verfahren zu einem Bereitstellen eines trainierten künstlichen neuronalen Netzes ausführen. Die zweite Recheneinheit weist ein Rechenmodul auf. Dieses Rechenmodul kann beispielweise für das Training des künstlichen neuronalen Netzes verwendet werden.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Die erste Recheneinheit und die zweite Recheneinheit entsprechen einer solchen programmierbaren Recheneinheit und weisen jeweils einen Speicher auf.

Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor der Recheneinheit geladen werden kann, der beispielsweise als Teil des Ultraschallsystems ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine erste Recheneinheit und/oder Planungseinheit und/oder Messeinheit des Ultraschallsystems und/oder zweite Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Merkmale, Vorteile oder alternative Ausführungsformen des erfindungsgemäßen Verfahrens zum Bestimmen eines Bewegungsdatensatzes durch Anwendung eines trainierten neuronalen Netzes auf eine medizinische Fragestellung und/oder des zugehörigen Ultraschallsystems und/oder des Computerprogrammprodukts können ebenso auch auf die jeweiligen anderen beanspruchten Gegenstände übertragen werden und umgekehrt.

Im Folgenden wird die Erfindung anhand der in den Figuren dargestellten Ausführungsbeispiele näher beschrieben und erläutert.

Es zeigen:

Fig. 1    ein Ultraschallsystem 11,

Fig. 2    ein Flussdiagramm eines erfindungsgemäßen Verfahrens zu einem Bereitstellen eines trainierten künstlichen neuronalen Netzes und

Fig. 3    ein Flussdiagramm eines erfindungsgemäßen Verfahrens zum Bewegen eines Roboterarms.

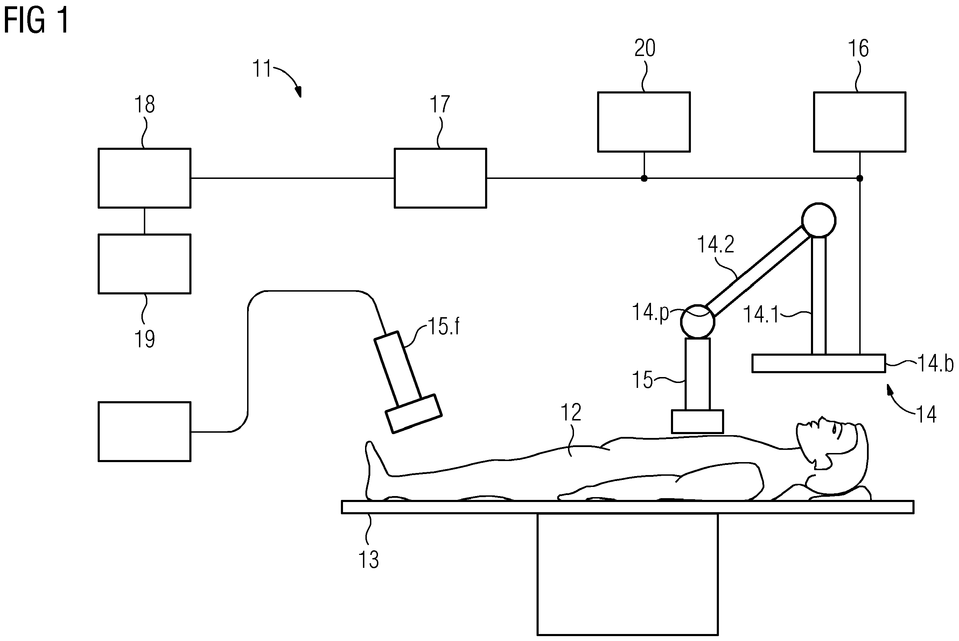

Fig. 1 zeigt ein Ultraschallsystem 11, wobei das Ultraschallsystem 11 einen Roboterarm 14 und einen Sensor, insbesondere eine Kamera 16 aufweist. An dem Roboterarm 14 ist ein Ultraschallkopf 15 befestigt. Der Roboterarm 14 ist vorzugweise derart ausgestaltet, dass eine Ultraschalluntersuchung eines Patienten 12 mit dem Roboterarm 14 durchführbar ist. Vorzugsweise ist der Patient 12 auf einer Patientenlagerungsvorrichtung 13, insbesondere auf einer Patientenliege gelagert. Üblicherweise liegt der Patient 12 supine auf der Patientenliege. Alternativ kann der Patient 12 auch sitzen während der Ultraschalluntersuchung.

Das Ultraschallsystem 11 umfasst eine Planungseinheit 17, eine erste Recheneinheit 18 und eine Messeinheit 19. Die Messeinheit 19 weist insbesondere den Roboterarm 14 für eine Ultraschalluntersuchung, wobei an dem Roboterarm 14 der Ultraschallkopf 15 befestigt ist, auf. Der Roboterarm 14 weist insbesondere eine Steuereinheit 20 des Roboterarms 14 und die Kamera 16 auf.

Ein freier Ultraschallkopf 15.f ist nicht mit dem Roboterarm 14 verbunden. Wenn der freie Ultraschallkopf 15.f mit dem Roboterarm 14 verbunden ist, dann entspricht der ehemals freie Ultraschallkopf 15.f dem Ultraschallkopf 15. Der freie Ultraschallkopf 15.f ist Teil eines separaten Ultraschallsystems.

Beispielsweise kann der freie Ultraschallkopf 15.f zum Bereitstellen von Trainingsbewegungsdatensätzen durch den Nutzer verwendet werden. Gemäß einer bevorzugten Ausführung kann der Nutzer zumindest eine Trainingsultraschalluntersuchung mittels des freien Ultraschallkopfes 15.f, welcher nicht an dem Roboterarm 14 befestigt ist, basierend auf der zumindest einen medizinischen Trainingsfragestellung, wobei der Nutzer den freien Ultraschallkopf 15.f bewegt, ausschließlich manuell durchführen. Dann werden die Bewegungen des freien Ultraschallkopfs 15.f während der zumindest einen Trainingsultraschalluntersuchung mittels eines Sensors, insbesondere der Kamera 16 erfasst und die erfassten Bewegungen in zumindest einen mittels des Roboterarms 14 durchführbaren Trainingsbewegungsdatensatz übersetzt.

Beispielsweise ist es auch denkbar, dass der Nutzer mit dem freien Ultraschallkopf 15.f eine erste Ultraschalluntersuchung durchführt und der Roboterarm 14 gemäß der ersten Ultraschalluntersuchung eine zweite Ultraschalluntersuchung durchführt. In anderen Worten, der Roboterarm 14 kann die erste Ultraschalluntersuchung zunächst erfassen und darauf vorzugweise wiederholen durch die Durchführung der zweiten Ultraschalluntersuchung.

Der Roboterarm 14 weist außerdem ein erstes Trägerelement 14.1 mit einer Länge L1 und ein zweites Trägerelement 14.2 mit einer Länge L2 auf. Das erste Trägerelement 14.1 ist an einer Aufhängevorrichtung 14.a angeordnet. Das zweite Trägerelement 14.2. ist mit dem ersten Trägerelement 14.1 verbunden. Die Aufhängevorrichtung 14.a ist scheibenförmig ausgebildet und in der Aufhängevorrichtung 14.a liegen eine x-Achse und eine y-Achse eines Koordinatensystems. Die z-Achse steht senkrecht auf einer Ebene, die von der x-Achse und der y-Achse des Koordinatensystems gebildet wird. Ein erster Winkel W1 beschreibt einen Winkel zwischen der x-Achse und der y-Achse. Ein zweiter Winkel W2 beschreibt einen Winkel zwischen einer sich in Längsrichtung des ersten Trägerelement 14.1 erstreckenden Längsachse und der z-Achse. Ein dritter Winkel W3 beschreibt einen Winkel zwischen der sich in Längsrichtung des ersten Trägerelement 14.1 erstreckenden Längsachse und einer sich in Längsrichtung des zweiten Trägerelement 14.2 erstreckenden Längsachse. Der Referenzpunkt 14.p entspricht einem Ende des zweiten Trägerelements 14.2, welches nicht mit dem ersten Trägerelement 14.1 verbunden ist.

Die Steuereinheit 20 erfasst beispielsweise den Bewegungsablauf mittels geeigneter Algorithmen. Die Position in (x,y,z)-Koordinaten des Referenzpunkt 14.p kann beispielsweise mit der folgenden Gleichung berechnet werden:

$$x_{14.p} = \cos(W1) \cdot \left( L2\sin(W2 + W3) + L1\sin(W2) \right)$$

$$y_{14.p} = \sin(W1) \cdot \left( L2\sin(W2 + W3) + L1\sin(W2) \right)$$

$$z_{14.p} = L2\cos(W2 + W3) + L1\cos(W2)$$

Zusätzlich kann vorzugsweise die Orientierung des Roboterarms 14 über geeignete Algorithmen bestimmt werden. Die Orientierung entspricht üblicherweise einem Raumwinkel des Roboterarms 14 und kann aus den Winkeln W1, W2 und W3 berechnet werden.

$$c = \frac{x^2 + y^2 + z^2 - \left(L_1\right)^2 - \left(L_2\right)^2}{2L_1L_2}$$

$$W1 = \tan^{-1}\left(\frac{y}{x}\right)$$

$$W2 = 90° - \tan^{-1}\left(\frac{z}{\sqrt{x_2 + y_2}}\right) + \tan^{-1}\left(\frac{L2\sqrt{1-c^2}}{L_1 + L_2 c}\right)$$

$$W3 = \tan^{-1}\left(\frac{\sqrt{1-c^2}}{c}\right)$$

Der Roboterarm 14 kann weitere nicht gezeigte Freiheitsgrade beispielsweise durch zumindest ein Drehgelenk oder zumindest eine Teleskopeinheit aufweisen.

Die Steuereinheit 20 kann den bestimmten Bewegungsdatensatz derart verarbeiten, dass der Roboterarm gemäß dem Bewegungsablauf des Roboterarms bewegt wird.

Das dargestellte Ultraschallsystem 11 kann selbstverständlich weitere Komponenten umfassen, die Ultraschallsysteme 11 gewöhnlich aufweisen. Ebenso können die einzelnen Komponenten, insbesondere der Roboterarm 14, der Ultraschallkopf 15, die Kamera 16, die Planungseinheit 17, die erste Recheneinheit 18, die Messeinheit 19 und die Steuereinheit 20 in anderer Beziehung zueinander stehen und/oder in eine übergeordnete Einheit integriert sein. Eine allgemeine Funktionsweise eines Ultraschallsystems 11 ist zudem dem Fachmann bekannt, so dass auf eine detaillierte Beschreibung der weiteren Komponenten verzichtet wird.

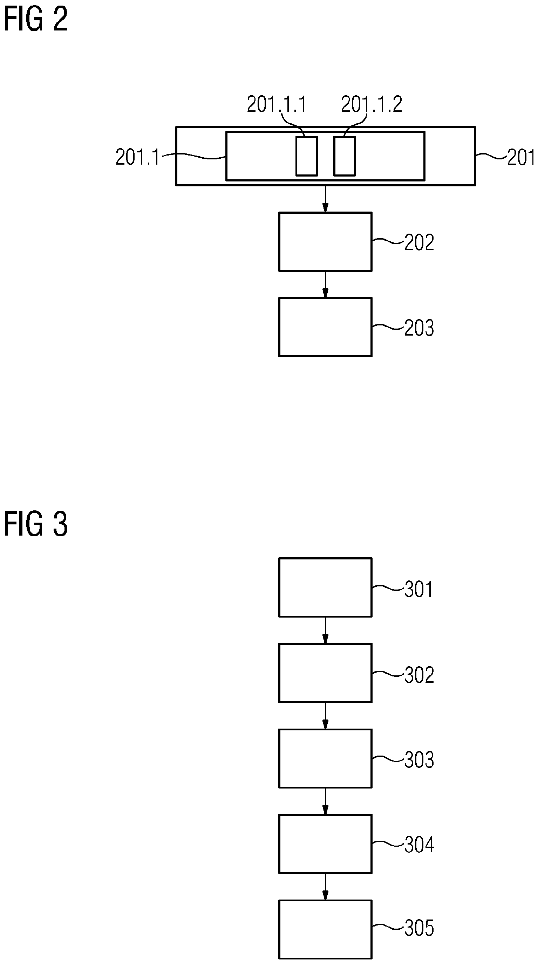

Das Ultraschallsystem ist vorzugweise außerdem dazu ausgebildet, die in Fig. 2 und Fig. 3 dargestellten Verfahrensschritte auszuführen.

Fig. 2 zeigt ein Flussdiagramm eines erfindungsgemäßen Verfahrens zu einem Bereitstellen eines trainierten künstlichen neuronalen Netzes für ein Bestimmen eines Bewegungsdatensatzes, welcher einen Bewegungsablauf eines Roboterarms 14 für eine Ultraschalluntersuchung, wobei an dem Roboterarm 14 ein Ultraschallkopf 15 befestigt ist. Das Verfahren umfasst die Verfahrensschritte 201-203 und die jeweiligen untergeordneten Verfahrensschritte.

Verfahrensschritt 201 kennzeichnet das Bereitstellen zumindest eines Trainingsbewegungsdatensatzes, wobei dem zumindest einen Trainingsbewegungsdatensatz zumindest eine medizinische Trainingsfragestellung zugeordnet ist.

Verfahrensschritt 202 kennzeichnet das Training eines künstlichen neuronalen Netzes unter Verwendung des zumindest einen Trainingsbewegungsdatensatzes und der zumindest einen medizinischen Trainingsfragestellung, wobei die Anwendung des trainierten künstlichen neuronalen Netzes auf die zumindest eine medizinische Trainingsfragestellung ein Bestimmen des zumindest einen Trainingsbewegungsdatensatzes ermöglicht. Verfahrensschritt 203 kennzeichnet das Bereitstellen des trainierten künstlichen neuronalen Netzes für ein Bestimmen des Bewegungsdatensatzes.

Untergeordneter Verfahrensschritt 201.1 kennzeichnet das Bereitstellen des zumindest einen Trainingsbewegungsdatensatz durch einen Nutzer. Das Bereitstellen 201.1 des zumindest einen Trainingsbewegungsdatensatzes durch den Nutzer weist folgende untergeordnete Verfahrensschritte auf:

- manuelles Durchführen 201.1.1 zumindest einer Trainingsultraschalluntersuchung mittels des Roboterarms (14) basierend auf der zumindest einen medizinischen Trainingsfragestellung,
- Erfassen 201.1.2 eines Trainingsbewegungsablaufs des zumindest einen Trainingsbewegungsdatensatzes während der zumindest einen manuell durchgeführten Trainingsultraschalluntersuchung.

Eine zweite Recheneinheit zu einem Bereitstellen eines trainierten künstlichen neuronalen Netzes umfasst ein Rechenmodul, wobei die zweite Recheneinheit zum Ausführen eines Verfahrens nach Verfahrensschritt 201-203 sowie untergeordnete Verfahrensschritte 201.1, 201.1.1 und 201.1.2 ausgebildet ist.

Die in Fig. 2 dargestellten Verfahrensschritte des erfindungsgemäßen Verfahrens werden von der zweiten Recheneinheit ausgeführt. Hierzu umfasst die zweite Recheneinheit erforderliche Software und/oder Computerprogramme und/oder ein Computerprogrammprodukt, die in einer Speichereinheit der zweiten Recheneinheit gespeichert sind. Die Software und/oder Computerprogramme und/oder das Computerprogrammprodukt umfassen Programmmittel, die dazu ausgelegt sind, das erfindungsgemäße Verfahren auszuführen, wenn das Computerprogramm und/oder die Software und/oder das Computerprogrammprodukt in der zweiten Recheneinheit mittels einer Prozessoreinheit der zweiten Recheneinheit ausgeführt wird.

Fig. 3 zeigt ein Flussdiagramm eines erfindungsgemäßen Verfahrens zum Bewegen eines Roboterarms 14 für eine Ultraschalluntersuchung, wobei an dem Roboterarm 14 ein Ultraschallkopf 15 befestigt ist. Das Verfahren umfasst die Verfahrensschritte 301-305.

Verfahrensschritt 301 kennzeichnet das Bereitstellen eines trainierten künstlichen neuronalen Netzes.

Verfahrensschritt 302 kennzeichnet das Erfassen einer medizinischen Fragestellung. Das Erfassen der medizinischen Fragestellung weist vorzugsweise auf, dass ein Sensor und/oder ein Nutzer mittels einer graphischen Benutzeroberfläche zumindest einen Parameter der medizinischen Fragestellung bereitstellen. Die medizinische Fragestellung weist zumindest einen Parameter der folgenden Liste auf:

- eine zu untersuchende Körperregion eines Patienten 12,
- einen Mode der Ultraschalluntersuchung,
- eine Frequenz der Ultraschalluntersuchung und
- eine Vorgabe für die Ultraschalluntersuchung.

Gemäß einer weiteren Ausführungsform weist die medizinische Fragestellung patientenspezifische Daten eines Patienten 12 auf und die patientenspezifischen Daten weisen zumindest einen Parameter der folgenden Liste auf:

- eine Körpergröße des Patienten 12,
- ein Gewicht des Patienten 12,
- einen Krankheitsbefund des Patienten 12 und
- einen medizinischen Bilddatensatz des Patienten 12.

In einer weiteren Ausführung weist der Bewegungsablauf des Bewegungsdatensatzes eine Funktion der Orientierung des Roboterarms 14 über die Zeit und eine Funktion der Position des Roboterarms 14 über die Zeit auf.

Vorzugweise weist der Roboterarm 14 einen Sensor auf und der Sensor weist zumindest eine der folgenden Varianten auf:

- einen elektro-optischen Sensor,
- eine Kamera 16,
- einen Abstandssensor,
- einen Lagesensor und
- einen Drucksensor.

Verfahrensschritt 303 kennzeichnet das Bestimmen eines Bewegungsdatensatzes, welcher einen Bewegungsablauf des Roboterarms 14 aufweist, durch Anwendung des trainierten künstlichen neuronalen Netzes auf die medizinische Fragestellung.

Verfahrensschritt 304 kennzeichnet das Übertragen des Bewegungsdatensatzes an eine Steuereinheit 20 des Roboterarms 14.

Verfahrensschritt 305 kennzeichnet das Bewegen des Roboterarms 20 gemäß dem Bewegungsablauf des Bewegungsdatensatzes. Gemäß einer bevorzugten Ausführung erfolgt das Bewegen des Roboterarms 14 gemäß dem Bewegungsablauf des Bewegungsdatensatzes automatisch und/oder durch einen Nutzer erfolgt und das Bewegen des Roboterarms 14 gemäß dem Bewegungsablauf des Bewegungsdatensatzes weist auf das Ermitteln einer Abweichung einer Bewegung des Roboterarms von dem Bewegungsablauf des Bewegungsdatensatzes beim Bewegen des Roboterarms 14 mittels des Sensors und das Gegensteuern durch die Steuereinheit 20 gemäß der ermittelten Abweichung.

Vorzugweise weist das Bewegen des Roboterarms 14 durch den Nutzer gemäß dem Bewegungsablauf des Bewegungsdatensatzes das Unterstützen des Nutzers beim Bewegen des Roboterarms 14 gemäß dem Bewegungsablauf des Bewegungsdatensatzes auf.

Die in Fig. 3 dargestellten Verfahrensschritte des erfindungsgemäßen Verfahrens werden von der ersten Recheneinheit 18 des Ultraschallsystems 11 ausgeführt. Hierzu umfasst die erste Recheneinheit 18 erforderliche Software und/oder Computerprogramme und/oder ein Computerprogrammprodukt, die in einer Speichereinheit der ersten Recheneinheit 18 gespeichert sind. Die Software und/oder Computerprogramme und/oder das Computerprogrammprodukt umfassen Programmmittel, die dazu ausgelegt sind, das erfindungsgemäße Verfahren auszuführen, wenn das Computerprogramm und/oder die Software und/oder das Computerprogrammprodukt in der ersten Recheneinheit mittels einer Prozessoreinheit der ersten Recheneinheit ausgeführt wird.

**Patentansprüche**

**1.** Verfahren zum Bewegen eines Roboterarms (14) für eine nicht invasive Ultraschalluntersuchung, wobei an dem Roboterarm (14) ein Ultraschallkopf (15) befestigt ist, umfassend folgende Verfahrensschritte:

- Bereitstellen eines trainierten künstlichen neuronalen Netzes,
- Erfassen einer medizinischen Fragestellung,
- Bestimmen eines Bewegungsdatensatzes, welcher einen Bewegungsablauf des Roboterarms (14) aufweist, durch Anwendung des trainierten künstlichen neuronalen Netzes auf die medizinische Fragestellung,
- Übertragen des Bewegungsdatensatzes an eine Steuereinheit (20) des Roboterarms (14) und
- Bewegen des Roboterarms (14) gemäß dem Bewegungsablauf des Bewegungsdatensatzes, **gekennzeichnet dadurch, dass** der Bewegungsablauf mittels eines Projektors auf eine Körperoberfläche eines Patienten projiziert wird.

2. Verfahren nach Anspruch 1, wobei das Erfassen der medizinischen Fragestellung aufweist, dass ein Sensor und/oder ein Nutzer mittels einer graphischen Benutzeroberfläche zumindest einen Parameter der medizinischen Fragestellung bereitstellen.

3. Verfahren nach einem der vorhergehenden Ansprüche, wobei die medizinische Fragestellung zumindest einen Parameter der folgenden Liste aufweist:

- eine zu untersuchende Körperregion eines Patienten (12),
- einen Mode der Ultraschalluntersuchung,
- eine Frequenz der Ultraschalluntersuchung und
- eine Vorgabe für die Ultraschalluntersuchung.

4. Verfahren nach einem der vorhergehenden Ansprüche, wobei die medizinische Fragestellung patientenspezifische Daten eines Patienten (12) aufweist und wobei die patientenspezifischen Daten zumindest einen Parameter der folgenden Liste aufweisen:

- eine Körpergröße des Patienten (12),
- ein Gewicht des Patienten (12),
- einen Krankheitsbefund des Patienten (12) und
- einen medizinischen Bilddatensatz des Patienten (12).

5. Verfahren nach einem der vorhergehenden Ansprüche, wobei der Bewegungsablauf des Bewegungsdatensatzes aufweist:

- eine Funktion der Orientierung des Roboterarms (14) über die Zeit und
- eine Funktion der Position des Roboterarms (14) über die Zeit.

6. Verfahren nach einem der vorhergehenden Ansprüche, wobei das Bewegen des Roboterarms (14) gemäß dem Bewegungsablauf des Bewegungsdatensatzes durch einen Nutzer erfolgt und das Bewegen des Roboterarms (14) durch den Nutzer gemäß dem Bewegungsablauf des Bewegungsdatensatzes aufweist:

- Unterstützen des Nutzers beim Bewegen des Roboterarms (14) gemäß dem Bewegungsablauf des Bewegungsdatensatzes.

7. Verfahren nach einem der vorhergehenden Ansprüche, wobei der Roboterarm (14) einen Sensor aufweist und der Sensor zumindest eine der folgenden Varianten aufweist:

- einen elektro-optischen Sensor,
- eine Kamera (16),
- einen Abstandssensor,
- einen Lagesensor und
- einen Drucksensor.

8. Verfahren nach Anspruch 7, wobei das Bewegen des Roboterarms (14) gemäß dem Bewegungsablauf des Bewegungsdatensatzes automatisch und/oder durch einen Nutzer erfolgt und das Bewegen des Roboterarms (14) gemäß dem Bewegungsablauf des Bewegungsdatensatzes aufweist:

- Ermitteln einer Abweichung einer Bewegung des Roboterarms von dem Bewegungsablauf des Bewegungsdatensatzes beim Bewegen des Roboterarms (14) mittels des Sensors und
- Gegensteuern durch die Steuereinheit (20) gemäß der ermittelten Abweichung.

9. Ultraschallsystem (11), umfassend

- eine Planungseinheit (17),
- eine erste Recheneinheit (18) und
- eine Messeinheit (19), welche einen Roboterarm (14) für eine Ultraschalluntersuchung, wobei an dem Roboterarm (14) ein Ultraschallkopf (15) befestigt ist, aufweist,

wobei das Ultraschallsystem dazu ausgebildet ist, ein Verfahren nach einem der vorhergehenden Ansprüche auszuführen.

10. Computerprogrammprodukt, welches direkt in einen Speicher einer programmierbaren Recheneinheit des Ultraschallsystems nach Anspruch 9 ladbar ist, mit Programmcode-Mitteln, um ein Verfahren nach einem der Ansprüche
1 bis 8 auszuführen, wenn das Computerprogrammprodukt in der Recheneinheit ausgeführt wird.

## Claims

1. Method for moving a robot arm (14) for a non-invasive ultrasound examination, wherein an ultrasound probe (15)
is attached to the robot arm (14), said method comprising the following method steps:

- providing a trained artificial neural network,
- recording a medical issue,
- determining a motion dataset containing a motion sequence of the robot arm (14) by applying the trained
artificial neural network to the medical issue,
- transferring the motion dataset to a control unit (20) of the robot arm (14), and
- moving the robot arm (14) in accordance with the motion sequence of the motion dataset, **characterised in
that** the motion sequence is projected onto the surface of the patient's body by means of a projector.

2. Method according to claim 1, wherein the recording of the medical issue comprises a sensor and/or a user providing
at least one parameter of the medical issue by means of a graphical user interface.

3. Method according to one of the preceding claims, wherein the medical issue includes at least one parameter from
the following list:

- a body region to be examined of a patient (12),
- a mode of the ultrasound examination,
- a frequency of the ultrasound examination, and
- a specification for the ultrasound examination.

4. Method according to one of the preceding claims, wherein the medical issue comprises patient-specific data of a
patient (12) and wherein the patient-specific data includes at least one parameter from the following list:

- a body height of the patient (12),
- a weight of the patient (12),
- a clinical finding on possible disorders of the patient (12) and
- a medical image dataset of the patient (12).

5. Method according to one of the preceding claims, wherein the motion sequence of the motion dataset includes:

- a function of the orientation of the robot arm (14) over time, and
- a function of the position of the robot arm (14) over time.

6. Method according to one of the preceding claims, wherein the moving of the robot arm (14) in accordance with the
motion sequence of the motion dataset is performed by a user and the moving of the robot arm (14) by the user in accordance with the motion sequence of the motion dataset comprises:

   - supporting the user during the moving of the robot arm (14) in accordance with the motion sequence of the motion dataset.

7. Method according to one of the preceding claims, wherein the robot arm (14) has a sensor and the sensor comprises at least one of the following variants:

   - an electrooptical sensor,
   - a camera (16),
   - a distance sensor,
   - a pose sensor, and
   - a pressure sensor.

8. Method according to claim 7, wherein the robot arm (14) is moved automatically and/or by a user in accordance with the motion sequence of the motion dataset and the moving of the robot arm (14) in accordance with the motion sequence of the motion dataset comprises:

   - determining a deviation of a movement of the robot arm from the motion sequence of the motion dataset during the moving of the robot arm (14) by means of the sensor, and
   - initiating countermeasures by the control unit (20) according to the determined deviation.

9. Ultrasound system (11) comprising

   - a planning unit (17),
   - a first computing unit (18), and
   - a measurement unit (19) which has a robot arm (14) for an ultrasound examination, wherein an ultrasound probe (15) is attached to the robot arm (14),

wherein the ultrasound system is embodied for performing a method according to one of the preceding claims.

10. Computer program product which can be loaded directly into a memory of a programmable computing unit of the ultrasound system according to claim 9 and has program code means for performing a method according to one of claims 1 to 8 when the computer program product is executed in the computing unit.

**Revendications**

1. Procédé de déplacement d'un bras (14) de robot pour un examen par ultrasons non-invasif, dans lequel une tête (15) à ultrasons est fixée au bras (14) du robot, comprenant les stades de procédé suivants :

   - on se procure un réseau neuronal artificiel ayant subi un apprentissage,
   - on détecte une position médicale en question,
   - on détermine un jeu de données de déplacement, qui a une évolution de déplacement du bras (14) du robot, en appliquant le réseau neuronal artificiel ayant subi un apprentissage à la position médicale en question,
   - on transmet le jeu de données de déplacement à une unité (20) de commande du bras (14) du robot et
   - on déplace le bras (14) du robot suivant l'évolution de déplacement du jeu de données de déplacement, **caractérisé en ce que** l'on projette l'évolution du déplacement au moyen d'un projecteur sur une surface du corps d'un patient.

2. Procédé suivant la revendication 1, dans lequel la détection de la position médicale en question s'effectue en ce qu'un capteur et/ou un utilisateur mette, au moyen d'une surface graphique d'utilisateur, à disposition au moins un paramètre de la position médicale en question.

3. Procédé suivant l'une des revendications précédentes, dans lequel la position médicale en question a au moins un paramètre de la liste suivante :

   - une région du corps à examiner d'un patient (12),

- un mode d'examen par ultrasons,
- une fréquence de l'examen par ultrasons et
- une prescription de l'examen par ultrasons.

4. Procédé suivant l'une des revendications précédentes, dans lequel la position médicale en question a des données d'un patient spécifiques au patient et dans lequel les données spécifiques au patient (12) ont au moins un paramètre de la liste suivante :

- une taille du patient (12),
- un poids du patient (12),
- une constatation de maladie du patient (12) et
- un jeu de données d'image médicale du patient (12).

5. Procédé suivant l'une des revendications précédentes, dans lequel l'évolution de déplacement du jeu de données de déplacement a :

- une fonction de l'orientation du bras (14) du robot en fonction du temps et
- une fonction de la position du bras (14) du robot en fonction du temps.

6. Procédé suivant l'une des revendications précédentes, dans lequel le déplacement du bras (14) du robot, suivant l'évolution du déplacement du jeu de données de déplacement, s'effectue par un utilisateur et le déplacement du bras (14) du robot par l'utilisateur, suivant l'évolution de déplacement du jeu de données de déplacement, a :

- une assistance de l'utilisateur lors du déplacement du bras (14) du robot, suivant l'évolution de déplacement du jeu de données de déplacement.

7. Procédé suivant l'une des revendications précédentes, dans lequel le bras (14) du robot a un capteur et le capteur a au moins l'une des variantes suivantes :

- un capteur électro-optique,
- une caméra (16),
- un détecteur de distance,
- un détecteur de position et
- un détecteur de pression.

8. Procédé suivant la revendication 7, dans lequel le déplacement du bras (14) du robot, suivant l'évolution de déplacement du jeu de données de déplacement, s'effectue automatiquement et/ou par un utilisateur et le déplacement du bras (14) du robot, suivant l'évolution de déplacement du jeu de données de déplacement, a :

- une détermination d'un écart d'un déplacement du bras (14) du robot à l'évolution de déplacement du jeu de données de déplacement lors du déplacement du capteur (14) du robot au moyen du capteur et
- une contre-commande par l'unité (20) de commande suivant l'écart déterminé.

9. Système (11) à ultrasons, comprenant

- une unité (17) de planification,
- une première unité (18) informatique et
- une unité (19) de mesure, qui a un bras (14) de robot pour un examen par ultrasons, dans lequel une tête (15) à ultrasons est fixée au bras (14) du robot,

dans lequel le système à ultrasons est constitué pour exécuter un procédé suivant l'une des revendications précédentes.

10. Produit de programme d'ordinateur, qui peut être chargé directement dans une mémoire d'une unité informatique programmable du système à ultrasons suivant la revendication 9, ayant des moyens de code de programme pour effectuer un procédé suivant l'une des revendications 1 à 8, lorsque le produit de programme d'ordinateur est réalisé dans l'unité informatique.

FIG 1

EP 3 409 230 B1

FIG 2

201.1.1    201.1.2

201.1    201

202

203

FIG 3

301

302

303

304

305

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9420997 B2
- WO 2017020081 A1
- US 20090088639 A1
- DE 102015212953 A1